# EUROPÄISCHE PATENTSCHRIFT

(11) **EP 1 374 865 B1**
(45) Veröffentlichungstag und Bekanntmachung des Hinweises auf die Patenterteilung: **08.06.2005**
(21) Anmeldenummer: 03018232.3
(22) Anmeldetag: 31.10.1995
(51) Int. Cl.: A61K 31/385, A61P 27/16, A61K 31/20

(54) **Verwendung von R,S(+/-)-alpha-Liponsäure, R-(+)-alpha-Liponsäure oder S-(-)-alpha-Liponsäure in reduzierter oder oxidierter Form zur Behandlung von Hörstörungen**
Use of R,S-(+/-)-alpha-lipoic acid, R(+)-alpha-lipoic acid or S-(-)-alpha-lipoic acid in reduced or oxidised form for the treatment of hearing disorders
Utilisation des acides R,S-(+/-)-alpha-lipoique, R-(+)-alpha-lipoique ou S-(-)-alpha-lipoique sous forme réduite ou oxydée pour le traitement de troubles auditifs

(30) Priorität: 08.11.1994 DE 4439477
(43) Veröffentlichungstag der Anmeldung: 02.01.2004
(62) Teilanmeldung aus: 95117108.1
(73) Patentinhaber: VIATRIS GmbH & Co. KG, 61352 Bad Homburg (DE)
(72) Erfinder: Conrad, Frank, Dr., 65933 Frankfurt (DE); Henrich, Herrmann-August, Prof.Dr., 97082 Würzburg (DE); Geise, Wolfgang, Dr., 97279 Prosselsheim (DE); Ulrich, Heinz Dr., 63843 Niedernberg (DE)

(56) Entgegenhaltungen:
- EP-A- 0 326 034
- EP-A- 0 661 051
- CHEMICAL ABSTRACTS, vol. 87, no. 17, 24. Oktober 1977 (1977-10-24) Columbus, Ohio, US; abstract no. 127616g, BORETS, VM ET AL.: "Evaluation of the effect of lipoic acid in patients with hypertensive and ischemic diseases" Seite 96; XP002946087 & BORETS MV ET AL.: "Evaluation of the effect of lipoic acid in patients with hypertensive and ischemic diseases" VOPR. PITAN., Bd. 4, 1977, Seiten 63-70,
- YADAV J S ET AL: "SYNTHESIS OF ALPHA-LIPOIC ACID-A HIGHLY USEFUL BIOLOGICALLY ACTIVE COMPOUND" JOURNAL OF SCIENTIFIC & INDUSTRIAL RESEARCH, XX, XX, Bd. 49, Nr. 8, 1. August 1990 (1990-08-01), Seiten 400-409, XP000645882
- NAUJOKS J ET AL: "DIE KONSERVATIVE THERAPIE DES TINNITUS EINE KURZE UEBERSICHT" LARYNGOLOGIE, RHINOLOGIE, OTOLOGIE, THIEME, STUTTGART, DE, Bd. 60, Nr. 4, April 1981 (1981-04), Seiten 220-223, XP001009436 ISSN: 0340-1588
- MARLOWE F: "DRUGS AND TINNITUS" HEARING INSTRUMENTS, HARCOURT BRACE JOVANOVICH PUBL. DULUTH, MINNESOTA, US, Bd. 40, Nr. 6, 1. Juni 1989 (1989-06-01), Seite 24,26 XP000115819 ISSN: 0092-4466
- SATALOFF R T: "TINNITUS: PROGRESS AND PROBLEMS IN RESEARCH" HEARING INSTRUMENTS, HARCOURT BRACE JOVANOVICH PUBL. DULUTH, MINNESOTA, US, Bd. 40, Nr. 6, 1. Juni 1989 (1989-06-01), Seite 22 XP000115817 ISSN: 0092-4466
- NIESCHALK M ET AL: "[Clinical aspects of coping with tinnitus]" LARYNGO- RHINO- OTOLOGIE. GERMANY OCT 1995, Bd. 74, Nr. 10, Oktober 1995 (1995-10), Seiten 594-600, XP008022945 ISSN: 0935-8943
- PREYER S ET AL: "[Tinnitus models for use in tinnitus counselling therapy of chronic tinnitus patients]" HNO. GERMANY JUN 1995, Bd. 43, Nr. 6, Juni 1995 (1995-06), Seiten 338-351, XP008022946 ISSN: 0017-6192
- MERZ P G ET AL: "ORALES ALPHA-LIPONSAUREPRAPARAT ERWEIST GUTE BIOVERFUGBARKEIT DIABETISCHE POLYNEUROPATHIE" THERAPIEWOCHE, KARLSRUHE, DE, Bd. 23, 1995, Seiten 1367-1370, XP000564932 ISSN: 0040-5973
- EHRENBERGER K ET AL: "REZEPTORPHARMAKOLOGISCHE MODELLE FUER EINE KAUSALE TINNITUSTHERAPIE-RECEPTOR PHARMACOLOGICAL MODELS FOR A CAUSAL TINNITUS THERAPY" OTO-RHINO-LARYNGOLOGIA, KARGER, PARIS, FR, Bd. 5, Nr. 3/4, 1. Mai 1995 (1995-05-01), Seiten 148-152, XP000609451 ISSN: 1014-8221

## Beschreibung

Die Erfindung betrifft die Verwendung von R,S(+/-)-alpha-Liponsäure, R-(+)-alpha-Liponsäure, S-(-)-alpha-Liponsäure in reduzierter oder oxidierter Form oder deren Metabolite sowie deren Salze, Ester, Amide zur Herstellung von Arzneimitteln zur Behandlung von Erkrankungen, die durch Veränderungen bzw. Störungen der rheologischen Bluteigenschaften wie der Blutviskosität, der Erythrozytenflexibilität und der Erythrozytenaggregation bedingt sind. Insbesondere ist sie einsetzbar bei Tinnitus und Hörsturz.

### Beschreibung

Bei Durchblutungsstörungen gewinnt die Therapie mit rheologisch wirksamen Substanzen an Bedeutung, besonders, wenn eine Rückbildung von rheologischen Hindernissen nicht mehr möglich ist. Rheologisch wirksame Substanzen verbessern die Fließfähigkeit des Blutes. Überwiegt neben anderen Wirkungen auf das Kreislaufsystem die rheologische Komponente werden diese durchblutungsfördernden Substanzen als "Rheologica" bezeichnet (Radke et al, 1983). Eine gesteigerte Fließfähigkeit erhöht den Blutfluß durch die Entstrombahn (Mikrozirkulation) und damit auch die Sauerstoffversorgung des Gewebes. Das ist besonders der Fall, wenn die Blutgefäße bei pathologischer Ausgangslage nicht mehr weiter gestellt werden können, also die vasomotorische Reserve erschöpft ist. Alpha-Liponsäure wird chemisch als 1,2-Dithiolan-3-Pentansäure 5-(1,2-dithiolan-3-yl)-Valeriansäure 5-3-(1,2-Dithioanyl)-Pentansäure bezeichnet. Die alpha-Liponsäure besitzt ein chirales C-Atom und tritt in zwei enantiomeren Formen auf und kommt physiologisch in Pflanzen, in Bakterien sowie im Säugerorganismus vor. Sie besitzt die Funktion eines Coenzyms in mitochondrialen Multienzymkomplexen wie z.B. dem der Pyruvatdehydrogenase, der alpha-Ketoglutaratdehydrogenase und der Dehydrogenase der verzweigtkettigen Aminosäuren. Im Stoffwechsel kann die alpha-Liponsäure von der oxidierten Form (Disulfidbrücke) in die reduzierte Dihydroform mit zwei freien SH-Gruppen überführt werden. Beide Formen haben eine ausgeprägte antioxidative Wirkung (z.B. Kuklinski et al., 1991; Packer, 1993). Das Redoxpaar Dihydroliponsäure / alpha-Liponsäure besitzt zudem metallchelatierende Eigenschaften. In der Bundesrepublik Deutschland wird alpha-Liponsäure seit 1966 als Arzneimittel zur Behandlung von Lebererkrankungen, bei Pilzvergiftungen sowie bei peripheren Polyneuropathien angewendet. Aus Kenntnis dieser antioxidativen Wirkung, wird in der DE-OS 4138040 A1 allgemein eine Verbindung die freie Radikale fängt und die Thiolfunktionen besitzt, genannt. Der Anspruch gilt für Lösungen für die Perfusion, Konservierung und Reperfusion von Organen und betrifft die hier vorliegende Erfindung nicht, weil über die bekannte antioxidative Eigenschaft hinaus hier eine neue Wirkung in einer neuen Anwendung gefunden wurde.
Das SU-Patent 1769865 A1 betrifft die Verwendung von u.a. alpha-Liponsäure zur phänomenologischen Verbesserung der Blutzirkulation und zwar die Vergrößerung des Blutstromes durch die großen Gebärmuttergefäße und die Verringerung der volumetrischen Parameter im Bereich des Chorions bei Plazentainsuffizienz.
Ein Kombinationspräparat mit Ginkgo biloba-Extrakt auch zur Prophylaxe und Behandlung von Durchblutungsstörungen ist in der EP-OS 0326 034 (Költringer ,1989) beschrieben.
Es bestand daher die Aufgabe bei Tinnitus und Hörsturz eine wirkungsvolle Therpiemöglichkeit zu schaffen.
Diese Aufgabe wurde erfindungsgemäß durch die Verbesserung des Blutflusses durch Verbesserung der Blutviskosität, die auf einer Erhöhung der Erythrozytenflexibilität und auf einer Verbesserung der pathologisch veränderten Erythrozytenaggregation bei den o.g. Krankheitsbildern durch Verabreichung von R,S-(+/-)-alpha-Liponsäure, R-(+)-alpha-Liponsäure, S-(-)-alpha-Liponsäure in reduzierter oder oxidierter Form sowie deren Salzen in Form von Arzneimitteln zur Prävention und Therapie von Erkrankungen, die durch Veränderungen bzw. Störungen der rheologischen Bluteigenschaften wie der Blutviskosität, der Erythrozytenflexibilität und der Erythrozytenaggregation bedingt sind, gelöst. Gegenstand der Erfindung ist demnach vor allem die Verwendung zur Herstellung von Arzneimitteln enthaltend R,S-(+/-)-alpha-Liponsäure, R-(+)-alpha-Liponsäure, S-(-)-alpha-Liponsäure in reduzierter oder oxidierter Form oder deren Salze, zur Prävention und Therapie bei Tinnitus und Hörsturz.

### Methode

Zum Nachweis der Wirksamkeit von alpha-Liponsäure und/oder ihren Enantiomeren, und/oder ihren Derivaten wurde die spezifische Methode der sinusoidalen oszillierenden Kapillar-Rheometrie verwendet (Chmiel H., 1990). Das viskoelastische Fließverhalten von Blut als modernstes Verfahren wird zur Bestimmung von pathologischen Veränderungen der Erythrozyten in der Klinischen Hämorheologie (z. B. bei arterieller Verschlußkrankheit, Schlaganfall und allgemein bei peripheren Gefäßkrankheiten) eingesetzt.
Zur Bestimmung der Viskoelastizität werden dynamisch rheologische Experimente durchgeführt, in denen zeitabhängig die Deformation und der Scher-Streß gemessen werden (sinusoidale oszillierende Scherversuche). Blut als nichtlineare viskoelastische Flüssigkeit zeigt eine Abnahme von η' und η" bei ansteigender Amplitude der Scherung.

Für die hier unter "Beispiele" aufgeführten Messungen wurde das Oszillierende Kapillar-Rheometer, OCR-D (A. Paar, Graz, Austria) verwendet, bei dem die Methode auf einer gleichzeitigen Bestimmung der Volumenflußrate und des Druckgradienten entlang einer kreisrunden Glaskapillare beruht. Die Viskoelastizität kann so zwischen elastischen (Energiespeicherung) und viskösen (Energieverbrauch) Deformationen unterscheiden. Ansteigende Werte von η" beschreiben zunehmend elastischere Erythrozyten (weniger flexibel) mit Bildung von Aggregaten, die zu Störungen des Blutflusses in der Mikrozirkulation führen. Diese Eigenschaften sind verknüpft mit der Beschaffenheit der Zellmembran und den "bridging"-Mechanismen, die zu der o.g. Rouleaux-Bildung führen. Die Abnahme von η' bei höheren Scherraten kann von veränderten Orientierungen und Verlängerung der Erythrozyten sowie von einer Reduzierung des Energieverbrauchs herrühren.

### 1.) Ex vivo-in vitro-Untersuchungen

Mit menschlichem Blut und dessen Erythrozytenkonzentraten wurden Untersuchungen zur Viskoelastizität an der Universitätsklinik Würzburg bei Herrn Prof. Dr. Henrich durchgeführt. Unter Lagerungsbedingungen wurde das Blut, versetzt mit alpha-Liponsäure, zeitabhängig aufbewahrt. Zu den verschiedenen Meßpunkten wurde das Blut im autologen Plasma resuspendiert und mit Phenazin-Methosulfat (PMS) versetzt, was die Ischämische Situation im Patienten nachempfindet (Methode: Maridonneau et al. 1983; Maridonneau-Parini und Harpey 1985).

### Die Dynamische Komponente der Blutyiskosität, η'

Beispielsweise bei Einsatz von beiden Enantiomeren der alpha-Liponsäure kommt es zu einer ausgeprägten, meist hochsignifikanten Verringerung der Blutviskosität gegenüber der Kontrolle. Der relative Unterschied liegt bei 10 %.

### Die Elastische Komponente der Blutviskosität. η"

Der relative Unterschied gegenüber der Kontrolle liegt hier bei 20 % Verbesserung.

### 2.) Tierexperimentelle Untersuchungen

Bei experimentellen Diabetes in Ratten wurde eine Verbesserung des perinervalen Blutflusses von Prof. Dr. Low, Mayo Clinic, Rochester, USA, gemessen. Die Durchblutung des peripheren Nervensystems der diabetischen Ratten war durch Thioctsäure um 50 % im Vergleich zur Kontrolle (nicht behandelte diabet. Tiere) gesteigert (p < 0,001).

### 3.) Untersuchung am Nagelfalz

7 Patienten mit diabetischer Polyneuropathie wurden 6 Wochen mit Thioctacid 2 x 600 behandelt. Es wurde am Anfang und am Ende eine kapillarmikroskopische Untersuchung am Nagelfalz durchgeführt. Im Rahmen der Untersuchung wurde ein Funktionstest durchgeführt, eine 3-minütige lschämie mit anschließender Reperfusion. Zielparameter war die Zeit bis zum Erreichen der Re-perfusionsgeschwindigkeit. Vor der Behandlung betrug die Zeit 76,8 ± 25,2 s, nach 6-wöchiger Behandlung 21,4 ± 8,1 s.

Die Herstellung der Salze erfolgt in bekannter Weise (siehe auch Patentschrift EP-A 427 247). Die pharmazeutischen Zubereitungen enthalten im allgemeinen 5 mg bis 3 g der erfindungsgemäß verwendeten Verbindungen als Einzeldosis. Die erreichten Wirkspiegel im Körper sollen nach Mehrfachgabe vorzugsweise zwischen 0,1 und 100 mg/kg Körpergewicht liegen. Die Verabreichung erfolgt in Form von Tabletten, Kapseln, Granulaten, Kautabletten, Lutschtabletten, Pillen, Dragees, Brausetabletten, Brausepulver, Fertigtrinklösungen, flüssigen Formen zur parenteralen Applikation. Fertigtrinklösungen und flüssige Formen zur parenteralen Applikation können alkoholische und wässrige Lösungen, Suspensionen und Emulsionen sein.
Bevorzugte Anwendungsformen sind zum Beispiel Tabletten, die vorzugsweise zwischen 5 mg und 2 g der erfindungsgemäß verwendeten Verbindungen sowie Lösungen, die vorzugsweise zwischen 1 mg und 200 mg der erfindungsgemäß verwendeten Verbindungen /ml Flüssigkeit enthalten.
Als Einzeldosen des Wirkstoffes sind beispielweise zu nennen:
a. orale Formen: 10 mg - 3 g
b, parenterale Formen (intravenös oder intramuskulär): 10 mg - 12 g

Die Dosen a) und b) können beispielweise 1 bis 6 mal täglich oder als Dauerinfusion gegeben werden.

### Beispiele zur Galenik

### Ausführungsbeispiele:

### Beispiel 1:

### Tabletten mit 100 mg R-(+)-alpha-Liponsäure

250 g R-(+)-alpha-Liponsäure werden mit 750 g mikrokristalliner Cellulose gleichmäßig verrieben. Nach dem Sieben der Mischung werden 250 g Stärke (Starch 1500/Colorcon), 732,5 g Lactose, 15 g Magnesiumstearat und 2,5 g hochdisperses Siliciumdioxid zugemischt und die Mischung zu Tabletten mit Gewicht 800,0 mg verpreßt.
Eine Tablette enthält 100 mg R-(+)-alpha-Liponsäure. Gegebenenfalls können die Tabletten nach üblichen Verfahren mit einem magensaftlöslichen oder magensaftpermeablen Filmüberzug versehen werden.

### Beispiel 2:

### Ampullen mit 250 mg R-(+)-alpha-Liponsäure als Trometamolsalz in 10 ml

250 g R-(+)-alpha-Liponsäure werden zusammen mit 352,3 g Trometamol (2-Amino-2-(hydroxymethyl)-1,3-propandiol) in einer Mischung aus 9 Liter Wasser für Injektionszwecke und 200 g 1,2-Propylenglykol unter Rühren gelöst. Die Lösung wird mit Wasser für Injektionszwecke auf 10 Liter aufgefüllt und anschließend durch ein Membranfilter der Porenweite 0,2 µm mit Glasfaservorfilter filtriert Das Filtrat wird unter aseptischen Bedingungen zu 10 ml in sterilisierte 10 ml Ampullen abgefüllt.
Eine Ampulle enthält in 10 ml Injektionslösung 250 mg R-(+)-alpha-Liponsäure als Trometamolsalz.

### Beispiel 3:

### Ampullen mit 250 mg R-(-)-Dihydroliponsäure in 10 ml Injektionslösung

60 mg Trometamol und 1 g Ethylendiamintetraessigsäure, Dinatriumalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g R-(+)-Dihydroliponsäure gelöst. Die Lösung wird mit Wasser für Injektionszwecke, das mit Stickstoff begast wurde, auf ein Volumen von 2 Liter aufgefüllt. Nach sorgfältigem Mischen wird die Lösung über einen Membranfilter der Porenweite 0,2 µm filtriert und das Filtrat unter aseptischen Bedingungen und unter Vor- und Nachbegasung mit Stickstoff in Ampullen zu 10 ml Füllvolumen abgefüllt.
Eine Ampulle enthält in 10 ml Lösung 250 mg R-(-)-Dihydroliponsäure als Trometamolsalz.

## Patentansprüche

1. Verwendung von R,S-(+/-)-alpha-Liponsäure, R-(+)-alpha-Liponsäure, S-(-)-alpha-Liponsäure, R,S-(-/+)-Dihydroliponsäure, R-(-)-Dihydroliponsäure oder S-(+)-Dihydroliponsäure zur Herstellung von Arzneimitteln zur Behandlung von Tinnitus und Hörsturz.

## Claims

1. Use of R,S-(+/-)-alpha-lipoic acid, R-(+)-alpha-lipoic acid, S-(-)-alpha-lipoic acid, R,S-(+/-)-dihydrolipoic acid, R-(-)-dihydrolipoic acid or S-(+)-dihydrolipoic acid for producing medicaments for the treatment of tinnitus and sudden loss of hearing.

## Revendications

1. Utilisation d'acide R,S-(+/-)-alpha-liponique, d'acide R-(+)-alpha-liponique, d'acide S-(-)-alpha-liponique, d'acide R,S-(-/+)-dihydroliponique, d'acide R-(-)-dihydroliponique ou d'acide S-(+)-dihydroliponique pour la préparation de médicaments pour le traitement du tinnitus et de la surdité brusque.
